(19) Europäisches Patentamt
European Patent Office
Office européen des brevets

(11) Veröffentlichungsnummer : **0 153 474 B1**

# (12) EUROPÄISCHE PATENTSCHRIFT

(45) Veröffentlichungstag der Patentschrift : **13.12.89**

(51) Int. Cl.$^4$ : **C 12 P 7/40**, C 12 P 41/00

(21) Anmeldenummer : **84115446.1**

(22) Anmeldetag : **14.12.84**

(54) **Verfahren zur Gewinnung von D-2-(6-Methoxy-2-naphthyl)-propionsäure.**

(30) Priorität : **16.12.83 DE 3345660**

(43) Veröffentlichungstag der Anmeldung : **04.09.85 Patentblatt 85/36**

(45) Bekanntmachung des Hinweises auf die Patenterteilung : **13.12.89 Patentblatt 89/50**

(84) Benannte Vertragsstaaten : **AT BE CH DE FR GB IT LI LU NL SE**

(56) Entgegenhaltungen :
**DE-A- 2 159 011**
**AGRICULTURAL BIOLOGICAL CHEMISTRY, Band 45, Nr. 6, 1981, Seiten 1389-1392, JP; IRIUCHIJIMA und KEIYU: "Asymmetric hydrolysis of -alpha-substituted carboxylic acid esters with microorganisms"**

(73) Patentinhaber : **BOEHRINGER MANNHEIM GMBH
Patentabteilung, Abt. E Sandhofer Strasse 112-132
Postfach 31 01 20
D-6800 Mannheim 31 Waldhof (DE)**

(72) Erfinder : **Maier, Josef
Schmädlstrasse 15
D-8120 Weilheim (DE)**
Erfinder : **Gloger, Manfred, Dr. rer. nat.
Karwendelstrasse 6
D-8120 Weilheim (DE)**
Erfinder : **v. Hoerschelmann, Detlev, Dr. phil. nat.
Fasanenweg 7
D-8121 Wielenbach (DE)**
Erfinder : **Strejcek, Jan, Dipl.-Ing.
Auf der Leiten 6 b
D-8127 Iffeldorf (DE)**
Erfinder : **Heimerl, Sebastian
Beringerweg 6
D-8132 Tutzing (DE)**

(74) Vertreter : **Huber, Bernhard, Dipl.-Chem. et al
Patentanwälte H. Weickmann, Dr. K. Fincke F.A. Weickmann, B. Huber Dr. H. Liska, Dr. J. Prechtel
Möhlstrasse 22 Postfach 860 820
D-8000 München 86 (DE)**

**EP 0 153 474 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Gewinnung von D-2-(6-Methoxy-2-naphthyl)-propionsäure folgender Formel :

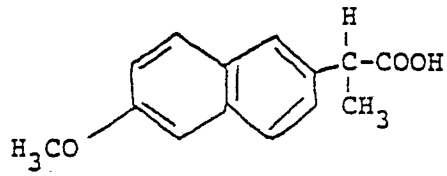

Diese Verbindung ist unter der Bezeichnung D(+)-Naproxen als Therapeutikum mit antiinflammatorischer Aktivität bekannt.

Es ist bekannt, die optisch reine D-Form aus der razemischen D,L-Mischung mit Hilfe von optisch aktiven Aminbasen, wie z. B. Chinchonidin, nach den üblichen Methoden der Razematentrennung zu gewinnen (DE-AS 2 159 011). Dort ist auch die chemische Spaltung von optisch aktiven Naproxenestern beschrieben, die durch 18 Stunden Erhitzen auf 50 °C mit Natriumhydroxid in Methanol in einer Ausbeute von 60 bis 70 % durchgeführt wird. Ferner ist es bekannt, das razemische Gemisch zu trennen, indem man den entsprechenden Methylester mit Hilfe von Aspergillus sojae (IAM 2031) selektiv spaltet, wobei bevorzugt die L(—)-Form des Esters gespalten wird (Agric. Biol. Chem. 45 (6), 1389-1392 (1981)). Diese mit schlechter Ausbeute (32 %) verlaufende Esterspaltung liefert die L(—)-Form, die Gewinnung der D(+)-Form wird nicht beschrieben. Der Erfindung liegt demgegenüber die Aufgabe zugrunde, ein Verfahren zur Gewinnung von D(+)-Naproxen zu finden, welches selektiver und einfacher als die bekannten Verfahren abläuft und eine praktisch vollständige Überführung des Razemats in die gewünschte D(+)-Form ermöglicht.

Gelöst wird diese Aufgabe erfindungsgemäß durch ein Verfahren zur Gewinnung von D-2-(6-Methoxy-2-naphthyl)-propionsäure aus Gemischen von D- und L-2-(6-Methoxy-2-naphthyl)-propionsäureestern von Alkylalkoholen mit 1 bis 4 Kohlenstoffatomen durch asymmetrische Hydrolyse des L-Esters mit einem mikrobiellen Enzym, Abtrennung der 2-(6-Methoxy-2-naphthyl)-propionsäure vom D-2-(6-Methoxy-2-naphthyl)-propionsäureester und Verseifung des letzteren, welches dadurch gekennzeichnet ist, daß man die Verseifung enzymatisch mit Esterase aus Schweineleber oder Pleurotus ostreatus durchführt.

Die Erfindung beruht auf der überraschenden Auffindung der Tatsache, daß die oben definierten Ester des Naproxens durch die Schweineleberesterase quantitativ gespalten werden und somit ein voll enzymatisches Verfahren ermöglicht, welches von den erwähnten Estern des razemischen Naproxens ausgehend, direkt zum gewünschten Produkt führt. Die gleiche Eigenschaft zeigt Esterase aus Pleurotus ostreatus. Esterasen anderer Herkunft erwiesen sich als ungeeignet. Erfindungsgemäß gewinnt man D(+)-Naproxen in einer optischen Reinheit von 98 %.

Die Schweineleberesterase oder Pleurotus ostreatus Esterase wird beim erfindungsgemäßen Verfahren vorzugsweise in immobilisierter Form eingesetzt. Für die Immobilisierung können die dem Fachmann bekannten Methoden der Trägerfixierung verwendet werden, beispielsweise Fixierung mit bromcyanaktivierten Trägern auf Basis von polymeren Kohlenhydraten wie Dextranen, Stärke, Cellulose und dergleichen, Vernetzung mit bifunktionellen Brückenbildnern wie Glutardialdehyd, Diepoxiden und dergleichen Adsorption an unlöslichen Trägern oder nach dem Verfahren der Enzymcopolymerisation, wie es z. B. in der DE-PS 21 28 743 und der DE-PS 22 60 185 beschrieben ist. Letzteres Verfahren wird bevorzugt. Schweineleberesterase ist bekannt und kann nach bekannten Methoden isoliert und gereinigt werden.

Die erste Stufe des erfindungsgemäßen Verfahrens kann in bekannter Weise unter Verwendung des Enzyms aus Aspergillus sojae, IAM 2 031, durchgeführt werden. Bessere Ergebnisse erhält man jedoch unter Verwendung einer spezifisch spaltenden Esterase aus Aspergillus oryzae, DSM 2808 (ATCC 11492), Aspergillus flavus, DSM 2807, Aspergillus sojae, DSM 2809 oder Bacillus subtilis, DSM 2806. Diese Enzyme geben eine bessere Selektivität der Spaltungsreaktion und weisen hohe Aktivität auf. Die Enzyme können in gereinigter Form oder auch in Form der kompletten Mikroorganismen eingesetzt werden. Insbesondere ist es möglich, das Mycel der genannten Aspergillusstämme direkt mit dem razemischen Estergemisch zu kontaktieren. Alternativ wird vorgereinigte Esterase verwendet und zwar zweckmäßig ebenfalls in trägerfixierter Form. Die obigen Angaben zur Trägerfixierung der Schweineleberesterase gelten in gleicher Weise für die mikrobielle Esterase der ersten Verfahrensstufe. Die Gewinnung der mikrobiellen Esterase erfolgt vorzugsweise durch Aufschluß des Mikroorganismus in üblicher Weise in Gegenwart eines Tensids bzw., mehr bevorzugt, nach Vorbehandlung mit einem Tensid. Die lösliche Fraktion des aufgeschlossenen Mikroorganismus kann dann nach üblichen Methoden, beispielsweise durch Chromatographie über einen schwach basischen Anionenaustauscher, vorgereinigt werden.

Der asymmetrischen Hydrolyse sind, wie schon erwähnt, die Ester des Napoxens von Alkoholen mit 1 bis 4 Kohlenstoffatomen zugänglich, nachstehend als Niedrigalkylester bezeichnet. Bevorzugt wird der Methyl- oder Ethylester verwendet.

Das bei der selektiven Hydrolyse anfallende Gemisch von D(+)-Naproxenester und L(—)-Naproxen läßt sich leicht trennen, indem man die Salze der Naproxensäure vom unlöslichen Ester quantitativ abtrennt. Die Trennung erfolgt am einfachsten durch Filtration oder Zentrifugation. Der so erhaltene reine D(+)-Ester wird dann mit der Schweineleberesterase oder Esterase aus Pleurotus ostreatus kontaktiert, wobei die freie Säure mit einer optischen Reinheit von etwa 98 % anfällt.

Gemäß einer bevorzugten Ausführungsform des Verfahrens der Erfindung erfolgt die Gewinnung des D(+)-Naproxens kontinuierlich, wobei man das razemische Estergemisch zuerst mit der mikrobiellen Esterase selektiv spaltet, den D(+)-Ester und die L(—)-Säure, vorzugsweise kontinuierlich, trennt, die abgetrennte L(—)-Säure razemisiert, das erhaltene Razemat in den Niedrigalkylester überführt und das razemische Estergemisch zur Spaltung mit mikrobieller Esterase recyclisiert. Der bei der Trennung von Ester und Säure in der Lösung verbliebene D(+)-Ester wird mit der Esterase aus Schweineleber oder Pleurotus ostreatus kontaktiert unter Freisetzung der D(+)-Säure ohne Veränderung der sterischen Konfiguration. Diese bevorzugte Ausführungsform des erfindungsgemäßen Verfahrens setzt die Enzyme in beiden Stufen zweckmäßig in immobilisierter Form ein. Besonders bewährt hat sich hierbei in der ersten Stufe die Verwendung von mit bifunktionellen Vernetzungsmitteln behandelten Aspergillus Mycel, während im Reaktor der zweiten Stufe bevorzugt die Esterase in der durch Enzymcopolymerisation erhaltenen unlöslichen Form eingesetzt wird.

Ein besonderer Vorteil dieser Ausführungsform der Erfindung besteht darin, daß durch die kontinuierliche Arbeitsweise unter Trennung von L-Säure und D-Ester nach der ersten Stufe der als Inhibitor für das Enzym der ersten Stufe wirkende abgespaltene Alkohol laufend entfernt wird und gleichzeitig hohe Substratkonzentrationen aufrechterhalten werden können, was die Gefahr vermeidet, daß gegen Ende der Reaktion durch die sinkende Substratkonzentration der L-Ester nicht mehr quantitativ gespalten wird.

Die Enzyme der ersten Stufe spalten die L-Form des Esters quantitativ, erfassen jedoch auch einen geringen Anteil des D-Esters. Durch die nachfolgende Razemisierung geht dieser D-Ester jedoch nicht verloren sondern wird erneut der Spaltung zugeführt, so daß man eine praktisch quantitative Umwandlung des razemischen Naproxens in D(+)-Naproxen erzielt.

Die Veresterung kann nach üblichen Methoden erfolgen, beispielsweise durch Erhitzen in dem jeweils gewählten Alkohol in Gegenwart eines sauren Katalysators wie Toluolsulfonsäure. Da der gebildete Ester im Alkohol schwer löslich ist, kann er leicht abgetrennt und in die Stufe der selektiven Esterspaltung eingeführt werden. Das Enzym der obengenannten bevorzugt verwendeten Aspergillen kann in Form des vollständigen, zweckmäßig mit difunktionellen Reagenzien wie Glutardialdehyd vernetzten Mycels oder in gereinigter Form, eingesetzt werden. Für die Reinigung erwies sich ein Aufschluß in Gegenwart eines oberflächenaktiven Mittels (Tensids) insbesondere nach Vorbehandlung mit diesem zweckmäßig. Geeignete Aufschlußmethoden sind beispielsweise Ultraschall, Vibrogenmühle, Dynomühle, Verreiben in der Reibschale oder Verwendung eines hochtourigen Mischers. Geeignete oberflächenaktive Mittel sind beispielsweise die Polyoxyethylenderivate wie Disorbitan-Fettsäureester, Sorbitanmonooleat, das Glycerinmonolaurat, Lanolinalkoholderivate, Sulfobernsteinsäurehalbester von oxyliertem Laurylalkohol, Laurylethersulfatsalze und dergleichen.

Das aus dem bevorzugten Mikroorganismus Aspergillus oryzae DSM 2808 (ATCC 11492) erhaltene Enzym weist ein Aktivitätsoptimum bei pH 8 auf und hat bei pH 5 noch etwa 65 % der Aktivität im pH-Optimum. Die $K_M$ wurde zu $2,2 \times 10^{-3}$ bestimmt. Die spezifische Aktivität im lyophilisierten Mycel beträgt etwa 5,5 U/g. Die Enzymaktivitäten werden mit dem Substrat D,L-Naproxenmethylester bei 25 °C und pH 7,8 im Autotitrator gemessen. Ein U entspricht dem Umsatz von 1 µMol/min.

Die folgenden Beispiele erläutern die Erfindung weiter:

## Beispiel 1

A) Herstellung des Esters

20 g razemisches Naproxen werden mit 0,5 g Toluolsulfonsäure in 200 ml Methanol 4 Stunden zum Rückfluß erhitzt. Nach Abkühlung auf 4 °C wird der gebildete Kristallbrei abgenutscht und mit wenig kaltem Methanol nachgewaschen. Ausbeute 19,5 g.

B) Selektive Esterspaltung

3,0 g lyophilisiertes Mycel von Asp. oryzae DSM 2808 (ATCC 11492), werden in 50 ml Wasser aufgeschlämmt und bis zur pH-Konstanz (7,8) gerührt.

Nach Zugabe von 1 ml Polyethylenglycol-Monobutylether werden 4 g des nach A) erhaltenen D,L-Naproxenmethylesters zugesetzt. Der pH-Wert wird durch Zudosierung von 0,2 m NaOH bei 7,8 konstant gehalten. Gebildetes L-Naproxen geht als Salz in Lösung und wird kontinuierlich durch Filtration aus der Reaktionslösung abgetrennt. Temperatur 40 °C.

Nach Beendigung der Umsetzung wird der D-Naproxenmethylester zweimal mit je 50 ml n-Pentan aus dem Reaktionsgemisch extrahiert, das Lösungsmittel abgedampft und der Rückstand in 50 ml Wasser resuspendiert.

500 mg Esterase aus Schweineleber (EC 3.1.1.1) werden in 40 ml Wasser gelöst und der Suspension des D-Naproxenesters zugesetzt. Dann wird die Suspension bei 32 °C und pH 7,2 bis zur vollständigen Spaltung gerührt. Zur Konstanthaltung des pH-Wertes werden insgesamt 9 ml 0,1 n NaOH zudosiert. Dann

wird mit 2 n HCl auf pH 1,5 eingestellt und das ausgefallene D-Naproxen abgetrennt. Der Niederschlag wird mit Ethanol extrahiert. Man erhält 1,52 g reines D(+)-Naproxen.

Beispiel 2

A) Züchtung von Asp. oryzae, DSM 2808 (ATCC 11492)

Medium :

0,5 % Pepton aus Casein (Merck)
0,5 % Pepton aus Fleisch (Merck)
2 % Glucose
1 % Maltose
pH 5.4 demineralisiertes Wasser

Fermentationsparameter :

| | |
|---|---|
| Temperatur | 28 °C |
| Drehzahl | 300 Upm |
| Belüftung | bei 10 l-Fermenter 300 l/h |
| pH-Statisierung | mind. pH 4,0 mit 10 % KOH |
| Antischaum | 10 % Wacker Silicon |

1. Vorkultur

Von Schraegagar in 5 ml Sabouraud-Medium (Rollkultur) 48 Stunden bei 28 °C bebrütet.

2. Vorkultur

Mit 1. Vorkultur 100 ml Sabouraud-Medium beimpft (500er Erlenmeyer mit 1 Schikane). Bebrütung 48 Stunden bei 28 °C und 180 Upm.

3. Vorkultur

Mit 2. Vorkultur 900 ml Sabouraud-Medium beimpft (2000er Erlenmeyer mit Magnetrührer). 70 Stunden bei 28 °C unter Rühren bebrütet.
Diese Vorkultur war geeignet, einen 10 l-Fermenter anzusetzen.

Züchtungsdauer :

96 bis 120 Stunden

Ernte :

Über Nutsche mit Gewebe mit 100 µm Porenweite.

Ausbeute :

870 g Feuchtmasse = 123 g Trockenmasse.

B) Herstellung trägerfixierter Esterase aus Asp. oryzae

Das nach A) gewonnene Mycel wird mit Polyoxyethylensorbitan-monooleat auf eine Endkonzentration von 0,04 % versetzt und nach 6 Stunden in der Hochdruckpresse (Manton/Gaulin) solubilisiert. Unlösliches wird abgetrennt und der Überstand über schwach basischen Kationenaustauscher (DEAE Sephadex G 50) chromatographiert. Die aktiven Fraktionen werden vereinigt und enthalten dann 6 U/ml.

5 ml dieser vorgereinigten Esterasepräparation werden mit 60 mg Methacrylsäure-N-Hydroxysuccinimidester, gelöst in 1,2 ml Dioxan, 30 Minuten bei 10 °C gerührt. Dann wird eine Lösung, bestehend aus 1,8 g Acrylamid, 0,3 g N,N-Methylen-bis-acrylamid, 15 ml 0,2 M TRAP-Puffer pH 8,0 zugesetzt. Nach Abkühlung auf 5 °C und Zugabe von 1 ml Ammoniumperoxidisulfat (10 %ig) und 1 ml 3-Dimethylaminopropionitril (10 %ig in Wasser) wird bis zum Einsetzen der Polymerisation mit Stickstoff begast.

Das erhaltene Gel wird nach 3 Stunden durch ein Sieb mit einer Maschenweite von 0,3 mm gepreßt und mit 0,5 M Phosphatpuffer, pH 7,5 und danach mit destilliertem Wasser gewaschen und lyophilisiert.

Man erhält trägergebundene Esterase mit einer spezifischen Aktivität von 3,6 U/g.

Bei Wiederholung des Verfahrens, jedoch ohne die 30 Minuten Vorinkubation, erhält man trägerfixierte Esterase mit einer spezifischen Aktivität von 1,8 U/g.

Das vorstehende Verfahren wird wiederholt unter Verwendung von Acrylsäurechlorid (30 µl in 1 ml Diethylether) anstelle des Methacrylsäure-N-hydroxysuccinimidesters. Die resultierende trägerfixierte Esterase besitzt eine spezifische Aktivität von 2,4 U/g.

C) Herstellung trägerfixierter Schweineleberesterase

Es wird, wie unter B) beschrieben, vorgegangen unter Verwendung von 40 ml Schweineleberesterase c = 10, 20 ml 0,2 M Tra/HCl Puffer pH 8,4 und 400 mg Methacrylsäure-N-hydroxysuccinimidester, gelöst in 2 ml Dioxan. Nach der 30 Minuten-Vorinkubation wird eine Lösung zugegeben aus 10 g Acrylamid, 2,5 g N,N-Methylen-bis-acrylamid, 40 ml 0,2 M Trap/HCl Puffer, pH 8, 4, 3 ml Ammoniumperoxidisulfat 20 %ig und 3 ml Dimethylaminopropionitrillösung 20 %ig und wie unter B) beschrieben, umgesetzt.

Die erhaltene trägerfixierte Schweineleberesterase besitzt eine spezifische Aktivität von 20 U/g.

D) Wie im Beispiel 1 beschrieben hergestellter razemischer Naproxen-Methylester wird mit einer Geschwindigkeit von 0,1 g/Std. in Form einer 2 %igen wässrigen Lösung von pH 7,8 einem 100 ml Reaktor zugeführt, der die nach B) erhaltene trägerfixierte Esterase aus Asp oryzae enthält. Der pH-Wert wird durch Zusatz von 0,2 n NaOH konstant gehalten. Gebildetes L-Naproxen-Natriumsalz wird kontinuierlich abfiltriert und nach Razemisierung und Veresterung wieder dem Reaktor zugeführt. Das filtrierte Reaktionsgemisch wird nach Abtrennung des trägerfixierten Enzyms einem zweiten Reaktor zugeführt, der 1 g der nach B) erhaltenen trägerfixierten Schweineleberesterase in 50 ml $H_2O$ suspendiert, erhält. Der pH-Wert wird durch Zudosierung von 0,2 n NaOH konstant bei 7,3 gehalten. Gebildetes D-Naproxen wird aus der Reaktionslösung mit 0,5 n HCl ausgefällt, zweimal gewaschen und getrocknet. Ausbeute : 98 %. $[\alpha]_D^{22} = 66,1$ (in Chloroform ; c = 0,5).

### Beispiel 3

Herstellung von Esterase (Extrakt) aus Pleurotus ostreatus (Austernseitling)

1 g Pleurotus ostreatus (getrocknet) werden zerkleinert, mit 50 ml 100 mM Phosphatpuffer pH 7,3 extrahiert, gegen 10 mM Phosphatpuffer pH 7,3 über 12 Stunden dialysiert und lyophilisiert.

Ausbeute 400 mg.

Dieser Extrakt kann anstatt der Esterase aus Schweineleber, wie in den Beispielen 1 und 2 näher beschrieben, verwendet werden.

## Patentansprüche

1. Verfahren zur Gewinnung von D-2-(6-Methoxy-2-naphthyl)-propionsäure aus Gemischen von D- und L-2-(6-Methoxy-2-naphthyl)-propionsäureestern von Alkylalkoholen mit 1 bis 4 Kohlenstoffatomen durch asymmetrische Hydrolyse des L-Esters mit einem mikrobiellen Enzym, Abtrennung der 2-(6-Methoxy-2-naphthyl)-propionsäure vom D-2-(6-Methoxy-2-naphthyl)-propionsäureester und Verseifung des letzteren, dadurch gekennzeichnet, daß man die Verseifung enzymatisch mit Esterase aus Schweineleber oder Pleurotus ostreatus durchführt.

2. Verfahren nach Anspruch 1, dadurch gekennzeichnet, daß man trägerfixierte Esterase aus Schweineleber oder Pleurotus ostreatus verwendet.

3. Verfahren nach Anspruch 1 oder 2, dadurch gekennzeichnet, daß man die asymmetrische Hydrolyse mit Esterase aus Aspergillus oryzae, DSM 2808 (ATCC 11492), Aspergillus flavus, DSM 2807, Aspergillus sojae, DSM 2809 oder Bacillus subtilis, DSM 2806 durchführt.

4. Verfahren nach Anspruch 3, dadurch gekennzeichnet, daß man den Mikroorganismus zuerst mit Tensid behandelt und dann aufschließt.

5. Verfahren nach einem der vorhergehenden Ansprüche, dadurch gekennzeichnet, daß man D-2-(6-Methoxy-2-naphthyl)-propionsäureniedrigalkylester und L-2-(6-Methoxy-2-naphthyl)-propionsäure kontinuierlich trennt, die L-2-(6-Methoxy-2-naphthyl)-propionsäure razemisiert, das gebildete Razemat verestert und das razemische Estergemisch zur Spaltung mit mikrobieller Esterase recyclisiert.

6. Verfahren nach einem der Ansprüche 3 bis 5, dadurch gekennzeichnet, daß man ein mit bifunktionellen Vernetzungsmitteln behandeltes Mycel von Aspergillus oryzae DSM 2808 (ATCC 11492) verwendet.

7. Verfahren nach Anspruch 5 und 6, dadurch gekennzeichnet, daß man einen pH-Wert zwischen 4 und 9, bevorzugt zwischen 7 und 8,5 aufrechterhält.

8. Verfahren nach Anspruch 5 bis 7, dadurch gekennzeichnet, daß man eine Temperatur zwischen 10 und 50, bevorzugt zwischen 30 und 45 °C aufrechterhält.

## Claims

1. Process for the obtaining of D-2-(6-methoxy-2-naphthyl)-propionic acid from mixtures of D- and L-2-(6-methoxy-2-naphthyl)-propionic acid esters of alkyl alcohols with 1 to 4 carbon atoms by asymmetric hydrolysis of the L-ester with a microbial enzyme, separation of the 2-(6-methoxy-2-naphthyl)-propionic acid from the D-2-(6-methoxy-2-naphthyl)-propionic acid ester and saponification of the latter, characterised in that one carries out the saponification enzymatically with esterase from hog liver or Pleurotus ostreatus.

2. Process according to claim 1, characterised in that one uses carrier-fixed esterase from hog liver or Pleurotus ostreatus.

3. Process according to claim 1 or 2, characterised in that one carries out the asymmetric hydrolysis with esterase from Aspergillus oryzae DSM 2808 (ATCC 11492), Aspergillus flavus DSM 2807, Aspergillus sojae DSM 2809 or Bacillus subtilis DSM 2806.

4. Process according to claim 3, characterised in that one first treats the micro-organism with tenside and then digests.

5. Process according to one of the preceding claims, characterised in that one continuously separates D-2-(6-methoxy-2-naphthyl)-propionic acid lower alkyl ester and L-2-(6-methoxy-2-naphthyl)-propionic acid, racemises the L-2-(6-methoxy-2-naphthyl)-propionic acid, esterifies the racemate formed and recycles the racemic ester mixture to the cleavage with microbial esterase.

6. Process according to one of claims 3 to 5, characterised in that one uses a mycelium from Aspergillus oryzae DSM 2808 (ATCC 11492) treated with a bifunctional cross-linking agent.

7. Process according to claim 5 and 6, characterised in that one maintains a pH value between 4 and 9, preferably between 7 and 8.5.

8. Process according to claim 5 to 7, characterised in that one maintains a temperature between 10 and 50 °C, preferably between 30 and 45 °C.

**Revendications**

1. Procédé pour l'obtention d'acide D-2-(6-méthoxy-2-naphtyl)-propionique à partir de mélanges d'esters d'acides D- et L-2-(6-méthoxy-2-naphtyl)-propioniques avec des alcools alcoyliques ayant de 1 à 4 atomes de carbone, par hydrolyse asymétrique du L-ester au moyen d'une enzyme microbienne, séparation de l'acide 2-(6-méthoxy-2-naphtyl)-propionique de l'ester d'acide D-2-(6-méthoxy-2-naphtyl)-propionique et saponification de ce dernier, caractérisé en ce qu'on effectue la saponification par voie enzymatique au moyen d'estérase provenant de foie de porc ou de Pleurotus ostreatus.

2. Procédé selon la revendication 1, caractérisé en ce qu'on utilise de l'estérase de foie de porc ou de Pleurotus ostreatus fixée sur un support.

3. Procédé selon la revendication 1 ou 2, caractérisé en ce qu'on effectue l'hydrolyse asymétrique au moyen d'estérase provenant de Aspergillus oryzae, DSM 2808 (ATCC 11492), Aspergillus flavus, DSM 2807, Aspergillus sojae, DSM 2809 ou Bacillus subtilis, DSM 2806.

4. Procédé selon la revendication 3, caractérisé en ce qu'on traite d'abord le microorganisme avec un agent tensioactif puis on le désagrège.

5. Procédé selon l'une des revendications précédentes, caractérisé en ce qu'on sépare en continu les esters d'alcoyle inférieur de l'acide D-2-(6-méthoxy-2-naphtyl)-propionique et l'acide L-2-(6-méthoxy-2-naphtyl)-propionique, on racémise l'acide L-2-(6-méthoxy-2-naphtyl)-propionique, on estérifie le racémate formé et on recycle le mélange d'esters racémique pour le clivage avec de l'estérase microbienne.

6. Procédé selon l'une des revendications 3 à 5, caractérisé en ce qu'on utilise un mycélium d'Aspergillus oryzae DSM 2808 (ATCC 11492), traité au moyen d'agents de réticulation bifonctionnels.

7. Procédé selon les revendications 5 et 6, caractérisé en ce qu'on maintient une valeur de pH entre 4 et 9, de préférence entre 7 et 8,5.

8. Procédé selon la revendication 5 à 7, caractérisé en ce qu'on maintient une température entre 10 et 50, de préférence entre 30 et 45 °C.